# EUROPÄISCHE PATENTSCHRIFT

(11) **EP 2 330 584 B1**
(45) Veröffentlichungstag und Bekanntmachung des Hinweises auf die Patenterteilung: **16.09.2015**
(21) Anmeldenummer: 10012666.3
(22) Anmeldetag: 01.10.2010
(51) Int. Cl.: G09B 23/28, A61B 1/12

(54) **Simulationssystem für das Training endoskopischer Operationen**
Simulation system for training endoscopic operations
Système de simulation pour l'entraînement d'opérations endoscopiques

(30) Priorität: 09.10.2009 DE 102009048994
(43) Veröffentlichungstag der Anmeldung: 08.06.2011
(73) Patentinhaber: Karl Storz GmbH & Co. KG, 78532 Tuttlingen (DE)
(72) Erfinder: Glöggler, Bernhard, 78532 Tuttlingen (DE); Speiser, Björn, 78628 Rottweil (DE); Hinding, Thomas, 78554 Aldingen (DE)

(56) Entgegenhaltungen:
- EP-A2- 1 638 064
- US-A1- 2005 196 739
- US-A1- 2007 238 929

## Beschreibung

Die vorliegende Erfindung betrifft ein Simulationssystem für das Training endoskopischer Operationen sowie ein Verfahren für das Training endoskopischer Operationen.

Vorrichtungen und Verfahren der genannten Art sind bekannt. So ist in der Offenlegungsschrift DE 10 2004 046 038 A1 ein virtueller OP-Simulator beschrieben, der insbesondere für das Training endourologischer Eingriffe vorgesehen ist. Im Gegensatz zu chirurgischen oder endoskopischen Trainingsmodellen, bei denen eine reale Nachbildung eines Körperteils bzw. eines körperinneren Hohlraums, in den die Spitze eines Endoskops eingeführt wird, zur Simulation eines chirurgischen bzw. minimal-invasiven Eingriffs genutzt wird, wird bei einem virtuellen Simulator mit den Methoden der "virtual reality" (virtuellen Realität) rechnergestützt ein interaktiv durch den Benutzer beeinflussbares Bild einer virtuellen Umgebung, insbesondere eines Körperhohlraums, erzeugt. Gemäß DE 10 2004 046 038 8 A1 umfasst der virtuelle OP-Simulator eine Simulationsrechnereinheit zur Erzeugung eines solchen virtuellen endoskopischen Bilds in Echtzeit, ein Instrument, dessen proximaler (d. h. dem Benutzer nahegelegener) Teil dem eines endoskopisch einsetzbaren Instruments nachgebildet ist, und eine Instrumenteneingabeeinheit zur Aufnahme des Instruments. Neben der Darstellung des virtuellen Bilds wird auch die Kraftrückwirkung auf das Instrument bzw. auf eine distal (d. h. benutzerfern) angeordnete Resektionsschlinge berechnet und dem Benutzer vermittelt. Das in die Instrumenteneingabeeinheit einzusetzende, einem Resektoskop nachgebildete Instrument weist eine Zulauf- und eine Ablaufleitung für Spülflüssigkeit auf. In den Leitungen sind jeweils Ventilhähne vorgesehen, um die Leitungen öffnen und schließen zu können. Diesen Ventilhähnen sind Mikropotentiometer zugeordnet, mit deren Hilfe die Bewegung der Ventilhähne erfasst wird. Die entsprechenden Signale werden an die Simulationsrechnereinheit übertragen und dort zur Berechnung des virtuellen Bildes ausgewertet.

Aus US 2005/0196739 A1 ist ein endoskopisches Simulationssystem bekannt, das ein für die Simulation speziell angepasstes Trainingsendoskop umfasst, sowie einen Detektor, der die vom Benutzer gesteuerten Bewegungen des distalen Endes eines flexiblen Schafts des Trainingsendoskops erfasst, eine Bilderfassungsvorrichtung, die die Form eines körperinneren Hohlorgans eines Patienten erfasst, und einen Bildprozessor, der aus den erfassten Daten ein virtuelles dreidimensionales Bild des Hohlorgans erzeugt. Am Bedienteil des Trainingsendoskops sind Knöpfe für Luft bzw. Wasserspülung sowie zum Absaugen vorhanden. Bei Betätigung der betreffenden Knöpfe werden deren Bewegungen erfasst und über eine elektrische Leitung an den Prozessor übermittelt.

Die Erfassung der Bewegung der Ventilknöpfe für Luft, Wasser und/oder Absaugen und die Umwandlung in von dem Bildrechner verwertbare Signale erfordert die Integration elektronischer bzw. elektromechanischer Elemente in das Trainingsendoskop. Üblicherweise sind bei einem flexiblen Endoskop die Betätigungselemente für Luft, Wasser und Saugen im Handgriff angeordnet. Dort ist jedoch praktisch kein Raum für die entsprechenden mechanischen und elektronischen Komponenten vorhanden. Hierbei ist zu berücksichtigen, dass für eine realitätsnahe Simulation der Handgriff des Trainingsendoskops bezüglich Größe, Form und Gewicht möglichst dem des Original-Endoskops entsprechen soll. Es ist daher konstruktiv sehr aufwendig, geeignete Sensoren in den Handgriff des Trainingsendoskops zu integrieren. Dies gilt umso mehr, wenn ein für chirurgische Eingriffe geeignetes Original-Endoskop für die Verwendung in einem derartigen Simulator anpasst werden soll.

Um möglichst realistische Trainingsbedingungen zu gewährleisten, ist bei einem Trainingsendoskop bzw. einer entsprechenden Umrüstung eines Original-Endoskops außerdem darauf zu achten, dass sich für den Benutzer auch an der Art und der Wirkung der Betätigung der Ventile, an der hierfür auszuübenden Kraft und an der spürbaren Rückwirkung der Ventilknöpfe möglichst wenig gegenüber dem klinischen einsetzbaren Endoskop ändern soll. Auch dies ist bei einer Integration von Sensoren zur Erfassung der Bewegung der Ventilknöpfe in das Trainingsendoskop nicht oder nur mit sehr hohem Aufwand in wünschenswertem Maße zu erreichen.

Bei vielen Endoskopen, insbesondere flexiblen Endoskopen, sind für die Steuerung der drei Funktionen Saugen, Insufflieren und Spülen nur zwei Ventilknöpfe vorgesehen, die auf die entsprechenden Leitungen einwirken, die von einem proximal angeordneten Anschluss an die entsprechenden Pumpen durch den Handgriff bis in den distalen Endbereich des Endoskops verlaufen. Durch Niederdrücken eines ersten Ventilknopfs wird ein erstes Ventil betätigt, das auf die Saugleitung einwirkt, indem es eine Verbindung zwischen einer Saugpumpe, die an einen proximalen Teil der Saugleitung angeschlossen ist, und einem distal des Ventils angeordneten distalen Teil der Saugleitung herstellt bzw. ganz oder teilweise unterbricht. Dementsprechend wird durch Drücken eines zweiten Ventilknopfs ein zweites Ventil betätigt, das eine Verbindung zwischen einem von einer Spülpumpe mit Druck beaufschlagten Spülflüssigkeitsbehälter, der an einen proximalen Teil der Spülleitung angeschlossen ist, und einem distal des Ventils angeordneten distalen Teil der Spülleitung herstellt bzw. ganz oder teilweise unterbricht. Das zweite Ventil weist ferner eine Öffnung auf, die mit dem proximalen und dem distalen Teil der Insufflationsleitung verbunden ist. Mit dem proximalen Teil der Insufflationsleitung ist eine Insufflationspumpe verbunden, die ein Insufflationsgas, beispielsweise Luft, unter einem geeigneten Überdruck bereitstellt. Solange die Öffnung offen ist, kann das Insufflationsgas durch die Öffnung entweichen, so dass sich kein wesentlich erhöhter Druck in der Insufflationsleitung aufbauen kann. Um Gas über den distalen Teil der Insufflationsleitung in eine Körperhöhle, in die das distale Ende des Endoskops eingesetzt ist, einzuleiten, verschließt der Benutzer die Öffnung mit einem Finger. Nun kann sich Druck in der Insufflationsleitung aufbauen, und das von der Insufflationspumpe gelieferte Gas wird in den distalen Teil der Insufflationsleitung und von dort in die Körperhöhle weitergeleitet. Durch gleichzeitiges Verschließen der Öffnung und teilweises Niederdrücken des zweiten Ventilknopfs kann gleichzeitig insuffliert und gespült werden. Auf diese Weise können die drei Funktionen Saugen, Insufflieren und Spülen mit Hilfe von zwei Ventilknöpfen gesteuert werden.

Für eine realistische Simulation muss das Niederdrücken der Ventilknöpfe detektiert und ausgewertet werden, ebenso wie das Verschließen der Öffnung im zweiten Ventilknopf. Während die Bewegung der Ventilknöpfe beim Niederdrücken beispielsweise durch Schalter oder Potentiometer detektiert werden kann, ist für die Erkennung des Verschließens der Öffnung ein Sensor notwendig, der das Vorhandensein des die Öffnung verschließenden Fingers detektiert. Hierfür könnte zwar im Prinzip beispielsweise eine Reflexionslichtschranke eingesetzt werden, jedoch kann auf diese Weise ein vollständiges nicht von einem unvollständigen Verschließen der Öffnung unterschieden werden. Ein realitätsnahes Training der Bedienung der Ventile ist bei der beschriebenen Anordnung von Ventilen auf diese Weise daher kaum möglich.

Es ist die Aufgabe der vorliegenden Erfindung, ein Simulationssystem für das Training endoskopischer Operationen sowie ein Verfahren für das Training endoskopischer Operationen anzugeben, wobei die o. g. Nachteile vermieden werden.

Diese Aufgabe wird durch ein Simulationssystem gemäß Anspruch 1 sowie durch ein Verfahren gemäß Anspruch 15 gelöst.

Ein erfindungsgemäßes Simulationssystem umfasst eine Endoskopvorrichtung, die ein Bedienteil mit mindestens einem Ventil aufweist. Das mindestens eine Ventil wirkt auf mindestens eine Leitung ein, die insbesondere eine Saugleitung, eine Insufflationsleitung und/oder eine Spülleitung sein kann. Das mindestens eine Ventil ist insbesondere durch einen Benutzer betätigbar, bevorzugt handbetätigbar. Es kann aber auch eine automatische oder eine indirekte, beispielsweise motorische und/oder über mechanische, elektrische und/oder elektronische Mittel vermittelte Betätigung, beispielsweise über ein Fußpedal oder auch eine Sprachsteuerung vorgesehen sein. In einer bevorzugten Ausführungsform weist das Simulationssystem eine Mehrzahl von insbesondere handbetätigbaren Ventilen auf, die auf eine Mehrzahl von Leitungen, insbesondere eine Saugleitung, eine Insufflationsleitung und eine Spülleitung, einwirken. In bevorzugter Weise entspricht die Endoskopvorrichtung einem proximalen Teil eines klinisch einsetzbaren Endoskops bzw. ist diesem nachgebildet und weist insbesondere einen Handgriff auf, der die bei einem klinisch einsetzbaren Endoskop vorhandenen Ventilknöpfe in der bei einem solchen Endoskop üblichen Weise trägt, die zur Betätigung von diesen zugeordneten Ventilen dienen, die auf eine Saugleitung, eine Insufflationsleitung und eine Spülleitung einwirken.

Weiterhin umfasst das Simulationssystem Druckerzeugungsmittel, die zur Beaufschlagung der mindestens einen Leitung mit Unter- oder Überdruck, bevorzugt mit Luft unter Unteroder Überdruck, angeordnet sind. Insbesondere können die Druckerzeugungsmittel jeweils mit einem proximalen Ende einer Saugleitung, einer Insufflationsleitung und einer Spülleitung verbunden sein. Dabei können jeweils unterschiedliche Drücke in der Saugleitung, in der Insufflationsleitung und in der Spülleitung erzeugt werden, die in bevorzugter Weise voneinander unabhängig sind, so dass das Verschließen oder Öffnen einer der Leitungen keine wesentliche Druckänderung in den beiden anderen Leitungen hervorruft.

Erfindungsgemäß sind Sensormittel vorgesehen zur Messung von Druck und/oder Fluss in der mindestens einen Leitung. Hierdurch wird durch jeweils mindestens einen Sensor die betreffende Messgröße in der betreffenden Leitung bestimmt. Insbesondere kann hierdurch der Druck bzw. der Fluss in einer Saugleitung, in einer Insufflationsleitung und in einer Spülleitung jeweils unabhängig voneinander gemessen werden.

Weiterhin sind Übertragungsmittel vorhanden zur Übertragung der von den Sensormitteln gelieferten Messwerte an eine Steuerungseinrichtung, die die Messwerte bei der Erzeugung eines virtuellen Bildes verwertet, das insbesondere eine endoskopische Ansicht eines körperinneren Hohlraums darstellt, in den das distale Ende eines Endoskops eingeführt worden ist. Die Steuerungseinrichtung umfasst insbesondere mindestens einen Prozessor zur Ausführung der hierfür notwendigen Rechenoperationen, wobei eine Vielzahl weiterer Daten und Parameter verwendet werden können. So kann beispielsweise eine Speichereinrichtung vorhanden sein zur Speicherung von Daten über den körperinneren Hohlraum, in dem eine chirurgische Operation stattfinden soll, die mit dem Simulationssystem trainiert werden soll. Diese Daten können ganz oder teilweise patientenspezifisch sein und können auch Daten über den Zugangsweg umfassen, so dass auch das Einführen des distalen Teils des Endoskops in den körperinneren Hohlraum simuliert werden kann. Ferner können weitere Parameter des Endoskops verarbeitet werden, die beispielsweise den Grad der Einfiihrung des Endoskopschafts in den Hohlraum, die Krümmung des Schafts, die vom Benutzer steuerbare Abwinkelung des distalen Endes, die Betätigung eines durch den Endoskopschaft eingeführten endoskopischen Arbeitsinstruments usw. darstellen können. Verfahren zur Erzeugung eines derartigen virtuellen Bildes sind unter dem Begriff "virtual reality" (virtuelle Realität) an sich bekannt, ebenso wie Rechnersysteme zur Durchführung solcher Verfahren; hierzu wird insbesondere auf die oben erwähnte DE 10 2004 046 038 A1 verwiesen.

Das von der Steuerungseinrichtung erzeugte, hier als "virtuelles Bild" bezeichnete Bild stellt die Szene dar, die bei Durchführung der entsprechenden endoskopischen Operation mit Hilfe der im Endoskop befindlichen optischen Systeme und einer dem Endoskop zugeordneten Kamera aufgenommen und für einen Benutzer, insbesondere den Arzt, der die chirurgische Operation durchführt, dargestellt würde. Hierzu umfasst das Simulationssystem eine Anzeigeeinrichtung zur Anzeige des von der Steuerungseinrichtung erzeugten virtuellen Bildes, beispielsweise einen Videoschirm, einen Videoprojektor oder eine am Kopf des Benutzer angeordnete Anzeigeeinrichtung (Head-Mounted Display). Das virtuelle Bild wird von der Steuerungseinrichtung bevorzugt in Echtzeit erzeugt und auf der Anzeigeeinrichtung dargestellt, um eine sofortige Rückwirkung der vom Benutzer vorgenommenen Manipulationen und dadurch eine realistische Simulation zu ermöglichen.

Die Betätigung eines Spülventils kann beispielsweise zur Darstellung eines Spülvorgangs ausgewertet werden, indem der Anstieg eines Flüssigkeitsspiegels im sichtbaren Volumen, beispielsweise in einem Bereich der Lunge oder des Abdomens, dargestellt wird. Liegt das Objektiv des Endoskops unterhalb des Flüssigkeitsspiegels, so kann die durch den Brechungsindex der Flüssigkeit verursachte Veränderung der räumlichen Wahrnehmung des Hohlraums und ggf. eine gewisse Trübung des Bilds dargestellt werden. Da es bei chirurgischen Eingriffen zu Blutungen kommen kann, die die endoskopische Sicht stark beeinträchtigen können, können diese auch Gegenstand der Simulation sein; in diesem Fall kann durch Betätigung des Spülventils wieder eine freiere Sicht erreicht werden.

Dementsprechend kann bei einer Betätigung eines Saugventils ein Absinken des Flüssigkeitsspiegels dargestellt werden. Initiiert der Benutzer eine Insufflation, so kann eine Vergrößerung des sichtbaren Volumens dargestellt werden. Umgekehrt kann es als Verringerung des Volumens der Körperhöhle dargestellt werden, wenn der Benutzer das insufflierte Gas wieder entweichen lässt bzw. das Saugventil betätigt. Bei gleichzeitiger Insufflation und Spülung kann auch beispielsweise das Aufsteigen von Gasblasen angezeigt werden, um eine möglichst realistische Simulation zu erreichen.

Die genannten Effekte, die von der Betätigung der Ventile für Saugen, Insufflation und Spülung ausgelöst werden, können jeweils durch weitere Parameter, wie beispielsweise den an einer Saug-, Spül- oder Insufflationspumpe eingestellten Druck, oder durch die Art des Insufflationsgases oder der Spülflüssigkeit beeinflusst werden. Ferner kann auch beispielsweise die Bewegung der Endoskopspitze, die der Benutzer über entsprechende Kontrollelemente steuern kann, oder die vom Benutzer gewählte Orientierung des Bedienteils relativ zu einer Längsachse des Endoskops bei der Berechnung des virtuellen Bilds berücksichtigt werden, beispielsweise für die Darstellung des Flüssigkeitsspiegels.

Dadurch, dass das Simulationssystem eine Endoskopvorrichtung umfasst, die mindestens eine Leitung, beispielsweise eine Saugleitung, eine Insufflationsleitung und/oder eine Spülleitung, sowie ein oder mehrere insbesondere vom Benutzer zu betätigende Ventile zur Einwirkung auf die mindestens eine Leitung aufweist, ferner Mittel zum Beaufschlagen der mindestens einen Leitung mit Unter- oder Überdruck, und Mittel zum Erfassen von Druck und/oder Durchfluss in der mindestens einen Leitung, wird eine realitätsnahe Simulation einer endoskopischen Operation ermöglicht. Insbesondere ist es dadurch nicht erforderlich, in dem räumlich sehr eingeschränkten Bereich der Ventile weitere Bauelemente anzuordnen, um die Bewegung der Ventilknöpfe zu detektieren, und es können Größe, Bauform und Gewicht des Handgriffs des Original-Endoskops bei einem Trainings-Endoskop unverändert übernommen oder bei einem Umbau eines klinisch einsetzbaren Endoskops ungeändert belassen werden. Ebenso sind auch keine zusätzlichen elektrischen Leitungen vom Handgriff zur Steuerungseinrichtung zur Übertragung von Messwerten der Bewegung der Ventilknöpfe erforderlich. Weiterhin wird erfindungsgemäß die Betätigung des mindestens einen Ventils durch die eigentliche Wirkung des Ventils detektiert, nämlich die Auswirkung auf die Druck- und/oder Flussverhältnisse in der mindestens einen Leitung, auf die eingewirkt wird, und nicht nur die Bewegung eines oder mehrerer Ventilknöpfe gemessen. Schließlich kann bei einem Endoskop, bei dem die Insufflation durch das Verschließen einer Ventilöffnung mit einem Finger gesteuert wird, das Verschließen der Öffnung selbst detektiert werden, und auch ein unvollständiges Verschließen der Öffnung kann sicher erkannt und in der Simulation verwendet werden. Auch hierdurch ist eine besonders realitätsnahe Simulation der Vorgänge bei einer endoskopischen Operation möglich.

Da sich durch die Betätigung des mindestens einen Ventils sowohl die Druckverhältnisse in einer Leitung ändern, auf die das Ventil einwirkt, als auch sich der Durchfluss des betreffenden Mediums durch die Leitung verändert, kann die Betätigung des Ventils sowohl mit Hilfe von Druck- als auch von Durchflusssensoren detektiert werden. Sowohl die Messwerte des Drucks als auch die Messwerte des Durchflusses durch die Leitung können deshalb für die Berechnung eines virtuellen Bilds, das die vom Benutzer gesteuerten Saug-, Insufflations- und/oder Spülvorgänge berücksichtigt, verwendet werden. Dabei können an der Saug-, Insufflations- und/oder Spülleitung jeweils ein oder mehrere Sensoren angeordnet sein, ebenso können auch gleichzeitig an einer Leitung Druck- und Durchflusssensoren oder an einer oder mehreren Leitungen Drucksensoren und an einer oder mehreren anderen Leitungen Durchflusssensoren verwendet werden. Die Verwendung von mehreren, insbesondere mehreren unterschiedlichen Sensoren ermöglicht dabei eine Erhöhung der Betriebssicherheit. Dabei können verschiedene Messprinzipien zum Einsatz kommen, beispielsweise als Drucksensoren integrierte mikromechanische Silizium-Sensoren.

Gemäß einer bevorzugten Ausführungsform werden Drucksensoren verwendet. Dies hat den Vorteil, dass zur Messung kein Eingriff in die Strömungsverhältnisse in den Leitungen erforderlich ist, insbesondere wird der Strömungswiderstand nicht verändert. Darüber hinaus sind Drucksensoren besonders einfach aufgebaut und kostengünstig erhältlich. In besonders bevorzugter Weise ist an der Saug-, Insufflations- und Spülleitung jeweils genau ein Drucksensor angeordnet, der von gleicher Bauart sein kann, wodurch eine besonders einfache und kostengünstige Realisierung möglich ist.

Um die Betätigung eines der Ventile zu detektieren, kann prinzipiell sowohl ein Unterdruck als auch ein Überdruck in der jeweiligen Leitung dienen. So können in einer Ausführungsform der Erfindung die Saug-, Insufflations- und Spülleitung alle mit Unterdruck oder alle mit Überdruck beaufschlagt werden, insbesondere mit einem gleich großen Druck. Dies hat den Vorteil einer besonders einfachen Ausfiihrung, insbesondere können an den proximalen Endbereichen der jeweiligen Leitungen gleichartige Druckerzeugungsmittel, beispielsweise gleiche Pumpen, oder auch eine einzige Pumpe oder ein einziger Druckbehälter angeschlossen werden, wodurch alle drei Leitungen mit Über- oder Unterdruck beaufschlagt werden.

In einer bevorzugten Ausführungsform der Erfindung sind die Druckerzeugungsmittel jedoch zur Erzeugung von Unterdruck in der Saugleitung und zur Erzeugung von Überdruck in der Insufflationsleitung und in der Spülleitung ausgebildet. Dies hat den Vorteil einer besonders realitätsnahen Simulation, da eine eventuelle Rückwirkung des in einer Leitung bestehenden Drucks auf die Betätigung des Ventils damit weitgehend der Realität entspricht. Auch beim Verschließen der Öffnung, die mit der Insufflationsleitung verbunden ist, mit einem Finger kann es für den Benutzer einen spürbaren Unterschied ausmachen, ob darin Über- oder Unterdruck herrscht. Für eine realistische Simulation ist es deshalb vorteilhaft, wenn die Insufflationsleitung mit Überdruck versorgt wird. Darüber hinaus können bei dieser Wahl der Druckverhältnisse die Leitungen in ähnlicher Weise ausgeführt sein wie bei einem Original-Endoskop, bei dem die Saugleitung für Unterdruck und die Spülund die Insufflationsleitung für Überdruck ausgebildet sind, und die letzteren beiden Leitungen im distalen Bereich des Endoskops zudem miteinander verbunden sein können. Zur Erzeugung des Über- bzw. Unterdrucks in den Leitungen können verschiedene Arten von Druckerzeugungsmitteln verwendet werden. So kann beispielsweise ein gemeinsames Druckreservoir jeweils über ein Druckminderungsventil mit mehreren Leitungen verbunden sein, oder es kann für jede der Leitungen ein Druckreservoir vorgesehen sein. Ebenso können zur Erzeugung des Drucks unterschiedliche Arten von Pumpen bzw. Kompressoren verwendet werden. Bevorzugt sind die Druckerzeugungsmittel derart ausgebildet, dass sich, soweit für den Benutzer erkennbar, die Endoskopvorrichtung möglichst genau so verhält wie ein entsprechendes Original-Endoskop, das an die entsprechenden, für den Einsatz bei endoskopischen Operationen vorgesehenen Geräte angeschlossen ist.

In einer bevorzugten Ausführungsform werden Pumpen, insbesondere Rollen- oder Membranpumpen, als Druckerzeugungsmittel eingesetzt. Dies hat den Vorteil, dass der jeweils benötigte Druck durch Einschalten der Pumpe zur Verfügung steht und nach Ausschalten der Pumpe nach kurzer Zeit wieder abgebaut ist, so dass für einen Umbau des Simulationssystems, beispielsweise zur Umrüstung auf ein anderes Trainings-Endoskop, keine besonderen Vorkehrungen getroffen werden müssen. Zusätzlich können weitere Druckerzeugungsmittel, wie etwa Druckbehälter und/oder Druckminderungsventile, vorgesehen sein. Die Verwendung von Rollen- oder Membranpumpen, insbesondere solcher für die Anwendung bei chirurgischen Operationen, hat den weiteren Vorteil, dass alle Einstellungen, der graduelle Druckaufbau beim Einschalten der Pumpe und die sonstige Handhabung bei der Benutzung des Simulationssystems weitgehend der Realität entsprechen. Dies gilt insbesondere auch für den allmählichen Druckauf- oder Druckabbau bei der Betätigung der Ventile.

Weiterhin ist es vorteilhaft, wenn mehrere Pumpen einen gemeinsamen Antrieb aufweisen. So können die Pumpen beispielsweise als Rollenpumpen ausgebildet sein, die jeweils eine Antriebswelle aufweisen, auf die ein gemeinsamer Antriebsmotor wirkt. Dies ermöglicht eine unabhängige Druckerzeugung, auch beispielsweise die Erzeugung von Unterdruck in einer Leitung und die Erzeugung von Überdruck in einer anderen Leitung, mit einem besonders geringen Aufwand.

In einer bevorzugten Ausführungsform sind die Sensoren analoge Sensoren und die Übertragungsmittel umfassen einen Verstärker und einen A/D-Wandler. Dies ermöglicht eine besonders kostengünstige Ausführung. Die Umwandlung der analogen Signale der Sensoren in digitale Signale ermöglicht die Verarbeitung durch einen digitalen Rechner, insbesondere durch den Rechner der Steuerungseinheit. Ferner ist es bevorzugt, wenn die digitalen Signale über eine USB-Schnittstelle übertragen werden. Eine solche Schnittstelle ist sehr verbreitet und ermöglicht insbesondere die Verwendung eines handelsüblichen PC (Personal Computer) zur Erfassung und Weiterverarbeitung der Signale der Sensoren.

Weiterhin ist es vorteilhaft, wenn die Druckerzeugungsmittel und die Sensormittel in einer Versorgungseinheit zusammengefasst sind. Diese kann ein kompaktes Gehäuse aufweisen, das Anschlüsse für die Saugleitung, die Insufflationsleitung und die Spülleitung aufweist, und in dem beispielsweise die Drucksensoren und die Pumpen, ggf. auch die Antriebe der Pumpen, aufgenommen sind. Ferner kann die Versorgungseinheit elektrische und elektronische Komponenten zur Übertragung der Messwerte der Sensoren an die Steuerungseinrichtung umfassen, wie beispielsweise Verstärker, A/D-Wandler und eine Schnittstelle zur Weiterleitung der Daten. Die Versorgungseinheit kann auch die Steuerungseinrichtung enthalten, was eine besonders kompakte Ausführung des Simulationssystems ermöglicht.

Gemäß einer besonders bevorzugten Ausführungsform ist die Endoskopvorrichtung mit der Versorgungseinheit lösbar verbunden. Dies hat den Vorteil einer besonders einfachen Handhabung, wenn das Simulationssystem beispielsweise auf eine andere, einem anderen Endoskoptyp nachgebildete Endoskopvorrichtung umgerüstet wird. Hierfür kann insbesondere an der Versorgungseinheit eine Buchse und an der Endoskopvorrichtung ein Stecker vorhanden sein, der in die Buchse der Versorgungseinheit eingesteckt wird. Eine besonders einfach zu realisierende Ausgestaltung ergibt sich, wenn Stecker und Buchse dem Anschluss des Original-Endoskops an eine Lichtquelle entsprechen; dieser kann auch einen Anschluss für eine Fluidleitung, beispielsweise für die Insufflationsleitung, umfassen. Auch das Gehäuse der Versorgungseinheit kann dem der für das betreffende Endoskop vorgesehenen Lichtquelle entsprechen. Ferner kann die Versorgungseinheit Anschlüsse oder Verbindungsleitungen aufweisen zum Verbinden mit dem proximalen Bereich der Saug-, Insufflations- und Spülleitungen der Endoskopvorrichtung. Die Versorgungseinheit kann wie die Lichtquelle auch eine Halterung für eine Spülflasche aufweisen, an die die Spülleitung der Endoskopvorrichtung angeschlossen wird und die mit der Versorgungseinheit zur Druckbeaufschlagung verbunden wird. Da diese Anordnung der beim klinischen Einsatz des Endoskops entspricht, hat dies den Vorteil einer besonders realistischen Simulation.

Gemäß einer weiteren bevorzugten Ausführungsform ist die Endoskopvorrichtung wie ein flexibles Endoskop mit einem Bedienteil, einem flexiblen, langerstreckten Schaft, und einem Versorgungsschlauch ausgebildet und weist ferner einen Sauganschluss, einen mit der Insufflationsleitung verbundenen Druckanschluss und einen Spülanschluss auf. Saug-, Druck- und Spülanschluss können am proximalen Ende des Versorgungsschlauchs angeordnet und zum Anschluss an Druckerzeugungsmittel ausgebildet sein, insbesondere zum Anschluss an eine Versorgungseinheit, die die Druckerzeugungsmittel umfasst. Der Spülanschluss ist insbesondere zum Anschluss eines Spülflüssigkeitsbehälters ausgebildet. In bevorzugter Weise ist das Bedienteil als Handgriff ausgebildet, das insbesondere ein Saugventil mit einem vom Benutzer betätigbaren Saugventilknopf aufweist und ein Spülventil mit einem vom Benutzer betätigbaren Spülventilknopf, der eine Öffnung besitzt, die mit der Insufflationsleitung verbunden ist und die für die Steuerung der Insufflation vom Benutzer mit einem Finger verschlossen werden kann. Das Bedienteil kann weitere Bedienelemente, etwa zur Steuerung der Kamera oder zur Steuerung des distalen Endbereichs des Endoskops, aufweisen. Diese Ausgestaltung ermöglicht eine besonders realistische Nachbildung eines klinisch einsetzbaren Endoskops.

Gemäß einer weiteren Ausführungsform ist die Endoskopvorrichtung ein klinisch einsetzbares Endoskop. Dieses weist die oben genannten Anschlüsse zum Anschluss an Saug-, Insufflations- und Spülpumpe bzw. an einen Spülflüssigkeitsbehälter auf, sowie einen Anschluss zum Anschließen an eine Lichtquelle, der auch den Anschluss für die Insufflationsleitung umfassen kann. Durch den Anschluss an die Versorgungseinheit kann daher ein Endoskop innerhalb des Simulationssystems verwendet werden, das hierfür nicht oder nur in einem geringen Maße modifiziert worden ist und für einen klinischen Einsatz geeignet ist. Hierdurch ist eine kostengünstige Realisierung bei einer besonders realistischen Simulation möglich.

Bei einem für den klinischen Einsatz geeigneten Endoskop ist die Saugleitung insbesondere mit einem Ventil verbunden, das in einer Position die Leitung öffnet, so dass Luft einströmen kann und sich in der Saugleitung allenfalls ein geringer Unterdruck ausbildet, der lediglich durch den Strömungswiderstand in dem kurzen proximalseitigen Teil der Saugleitung bestimmt wird. Bei Betrieb der Saugleitung mit Überdruck kann sich dementsprechend in dieser Stellung kein wesentlicher Überdruck in der Leitung ausbilden. Bei Betätigung des Saugventils wird diese Öffnung verschlossen und gleichzeitig eine Verbindung zum distalen Teil der Saugleitung des Endoskops hergestellt. Diese weist aufgrund der Länge und des geringeren Durchmessers einen gewissen Strömungswiderstand auf, so dass sich in der Saugleitung ein messbarer Unter- bzw. Überdruck ausbilden kann. Dieser wird gemessen und zur Erkennung der Betätigung des Saugventils verwendet. In ähnlicher Weise ist der Durchfluss durch die Saugleitung von der Betätigung des Ventils abhängig und kann zusätzlich oder alternativ hierzu gemessen und zur Erkennung der Betätigung des Saugventils verwendet werden.

Weiterhin ist bei einem für den klinischen Einsatz geeigneten Endoskop die Spülleitung insbesondere mit einem Ventil verbunden, das in einer Position die Leitung verschließt, so dass sich in der Spülleitung der von den Druckerzeugungsmitteln eingekoppelte Druck ausbildet. In einer anderen Position des Ventils wird eine Verbindung zum distalen Teil der Spülleitung hergestellt. Hierdurch kann das in der Spülleitung enthaltene Fluid austreten; dies ist bevorzugt Luft, kann aber auch zum Zweck einer besonders realistischen Simulation Spülflüssigkeit sein, insbesondere Wasser, ggf. mit Zusätzen. Dadurch, dass das Fluid austritt, tritt eine Druckänderung ein, die messbar ist und zur Erkennung der Betätigung des Spülventils verwendet werden kann. In analoger Weise kann der hierdurch ermöglichte Durchfluss des Fluids durch die Spülleitung gemessen und zusätzlich oder alternativ hierzu gemessen und zur Erkennung der Betätigung des Spülventils verwendet werden.

Die Insufflationsleitung ist bei einem für den klinischen Einsatz geeigneten Endoskop insbesondere mit einer Öffnung im Spülventil verbunden, die zur Einleitung des Insufflationsgases in eine Körperhöhle mit einem Finger verschlossen wird. Während sich bei unverschlossener Öffnung allenfalls eine durch den Strömungswiderstand im proximalen Teil der Insufflationsleitung bestimmte, geringe Druckdifferenz zum Umgebungsdruck einstellt, kann durch Verschließen der Öffnung aufgrund des Strömungswiderstands im distalen Teil der Insufflationsleitung eine deutliche Druckdifferenz zur Umgebung entstehen, die zur Erkennung des Verschließens der Öffnung verwendet werden kann. In analoger Weise kann die entsprechende Änderung des Durchflusses durch die Insufflationsleitung zusätzlich oder alternativ hierzu gemessen und zur Erkennung der Insufflation verwendet werden.

Wenn die distalen Öffnungen der Saugleitung und/oder der Insufflationsleitung verschließbar sind, kann hierdurch die jeweilige Druck- bzw. Flussdifferenz beim Betätigen des betreffenden Ventils vergrößert werden. Hierfiir kann beispielsweise eine Verschlusskappe auf das distale Ende des Endoskopschafts aufgesetzt werden, die beide Öffnungen verschließt. Dies ermöglicht eine sicherere Erkennung der Betätigung des jeweiligen Ventils.

Aufgrund der in dieser Weise festgestellten Betätigungen werden die entsprechenden Insufflations-, Saug- und Spülvorgänge in dem von der Steuerungseinrichtung erzeugten virtuellen Bild dargestellt, so dass in vorteilhafter Weise ein für den klinischen Einsatz geeignetes Endoskop innerhalb des Simulationssystems verwendet werden kann.

Gemäß einer anderen Ausführungsform ist die Endoskopvorrichtung ein Trainingsendoskop, das für den Einsatz im Simulationssystem modifiziert worden ist. Dies hat den Vorteil, dass das Trainingsendoskop nicht die gleichen Sicherheitsbestimmungen erfüllen muss wie ein klinisch einsetzbares Endoskop und kostengünstig verfügbar ist.

Gemäß einer weiteren bevorzugten Ausführungsform der Erfindung sind die Sensormittel in den Anschlüssen des Endoskops angeordnet. Dies hat den Vorteil, dass die Endoskopvorrichtung in der gleichen Weise mit einer Saugpumpe, einer Insufflationspumpe und einer Spülpumpe bzw. einem Spülwasserbehälter verbunden werden kann wie ein Original-Endoskop, so dass für den Simulationsbetrieb keine Modifikation der Saugpumpe, der Insufflationspumpe und der Spülpumpe bzw. des Spülwasserbehälters erforderlich ist. Zur Übertragung der Messwerte an die Steuerungseinrichtung kann in diesem Fall ein Kabel vorgesehen sein, das an die Endoskopvorrichtung angeschlossen wird, oder es können drahtlose Übertragungsmittel vorhanden sein.

Weiterhin ist es vorteilhaft, wenn die Saugleitung distalseitig des auf die Saugleitung einwirkenden Ventils und die Insufflationsleitung distalseitig des auf die Insufflationsleitung einwirkenden Ventils verschließbar oder verschlossen ist. In diesem Fall wird bei Betätigung des Saugventils bzw. Verschließen der Öffnung der Insufflationsleitung der Durchfluss ermöglicht bzw. unterbrochen, so dass eine besonders große Durchfluss- und Druckänderung in der betreffenden Leitung erzielt wird, die eine besonders sichere Erkennung der Betätigung des betreffenden Ventils ermöglicht.

Ferner ist es vorteilhaft, wenn die Spülleitung distalseitig des Bedienteils offen ist. Die Spülleitung wird dadurch beim Betätigen des Spülventils geöffnet, wobei der Strömungswiderstand des übrigen distalen Teils der Leitung entfällt. Auch hierdurch ändert sich bei der Betätigung des Ventils der Druck bzw. der Durchfluss in der Spülleitung um einen größeren Betrag, der eine besonders sichere Erkennung der Betätigung des betreffenden Ventils ermöglicht.

Ein erfindungsgemäßes Verfahren für das Training endoskopischer Operationen umfasst folgende Schritte:
- Bereitstellen einer Endoskopvorrichtung, die ein Bedienteil mit mindestens einem Ventil, bevorzugt einer Mehrzahl von Ventilen aufweist, und die weiterhin mindestens eine Leitung, insbesondere eine Saugleitung, eine Insufflationsleitung und/oder eine Spülleitung, aufweist, auf die das mindestens eine Ventil einwirkt,
- Beaufschlagen der mindestens einen Leitung mit Unter- oder Überdruck,
- Erfassen einer, insbesondere von einem Benutzer veranlassten, Betätigung des mindestens einen Ventils durch Messung von Druck und/oder Fluss in der mindestens einen Leitung,
- Übertragen der Messwerte von Druck und/oder Fluss an eine Steuerungseinrichtung,
- Erzeugen eines virtuellen Bildes einer endoskopischen Operationsszene durch die Steuerungseinrichtung in Abhängigkeit von der durch die Messwerte erfassten Betätigung der Ventile, und
- Anzeigen des virtuellen Bildes für den Benutzer.

Das erfindungsgemäße Verfahren kann weitere Schritte umfassen, wie etwa das Erfassen der Betätigung weiterer, ggf. vorhandener Bedienelemente, die beispielsweise die Einstellung einer Kamera und einer Lichtquelle oder die Abwinkelung des distalen Endbereichs des Endoskops betreffen bzw. simulieren, und die Verwendung der betreffenden Daten bei der Erzeugung des virtuellen Bildes für den Benutzer. Ebenso können beispielsweise Warnsignale bei Fehlbedienungen, beispielsweise bei Über- oder Unterschreiten eines in der simulierten Operationssituation geeigneten bzw. physiologisch verträglichen Drucks und/oder Durchflusses erzeugt und dem Benutzer visuell oder akustisch angezeigt werden.

Das erfindungsgemäße Simulationssystem und das erfindungsgemäße Verfahren sind für das Training endoskopischer Operationen beschrieben worden, wobei der Begriff "Operationen" auch beispielsweise diagnostische Anwendungen bzw. Eingriffe umfassen soll. Ebenso ist das erfindungsgemäße System wie auch das erfindungsgemäße Verfahren für Demonstrations- und Lehrzwecke geeignet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 ein Ausführungsbeispiel eines erfindungsgemäßen Simulationssystems in einer teilweise schematischen Übersichtsdarstellung;
Fig. 2 das Saugventil gemäß Ausführungsbeispiel in vergrößerter Ansicht in der eingedrückten Stellung;
Fig. 3 das Spül- und Insufflationsventil gemäß Ausführungsbeispiel in vergrößerter Ansicht in der eingedrückten Stellung;
Fig. 4 eine schematische Darstellung der Erfassung und Übertragung der Daten der Sensoren gemäß Ausführungsbeispiel.

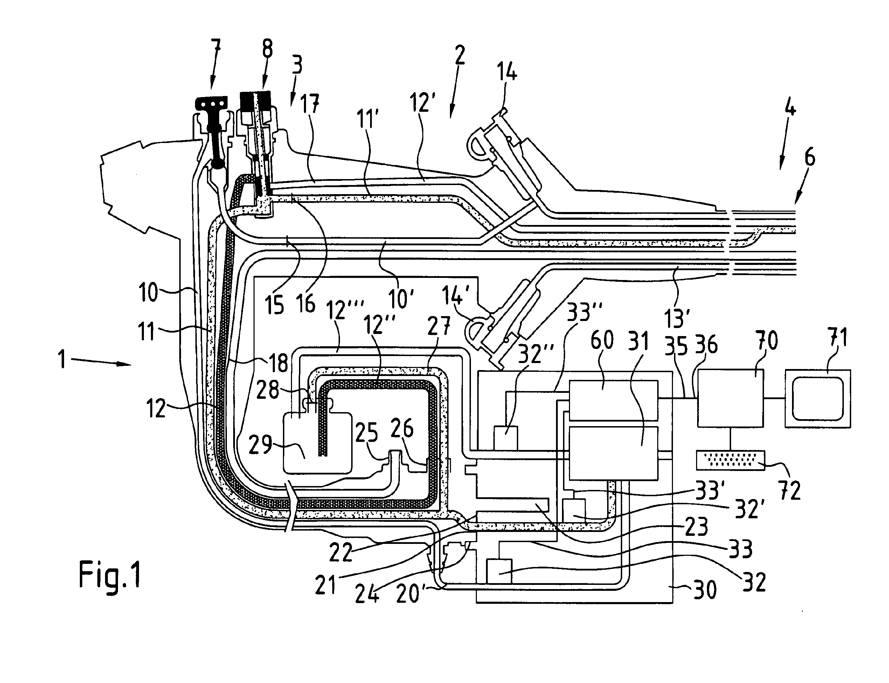

Wie in Fig. 1 gezeigt, besteht ein Ausführungsbeispiel des erfindungsgemäßen Simulationssystems 1 aus einer Endoskopvorrichtung 2, einer Versorgungseinheit 30, einer Steuerungseinrichtung 70, einer Anzeigeeinrichtung 71 sowie ggf. weiteren Komponenten, von denen in Fig. 1 beispielhaft eine Eingabeeinrichtung 72 und ein Spülflüssigkeitsbehälter 29 dargestellt sind. Die Endoskopvorrichtung 2 umfasst einen Handgriff 3, einen für die Einführung in eine Körperhöhle vorgesehenen langerstreckten flexiblen Schaft 4 und einen Versorgungsschlauch 5. Am Handgriff 3 sind ein Saugventil 7 und ein Spül- und Insufflationsventil 8 angeordnet. Die in Fig. 1 dargestellte Endoskopvorrichtung 2 ist als Trainingsendoskop ausgebildet, das durch Modifikation aus einem klinisch einsetzbaren Original-Endoskop hervorgegangen ist.

Das Saugventil 7 dient zum Steuern des Absaugens aus dem Operationsgebiet. Hierzu ist in der Endoskopvorrichtung proximalseitig des Saugventils 7 eine Saugleitung 10 vorgesehen, auf die das Saugventil 7 einwirkt. Distalseitig des Saugventils 7 ist ebenfalls eine Saugleitung 10' vorgesehen, die bei einem Original-Endoskop weiter durch den Endoskopschaft 4 bis zum distalen Ende 6 durchgeführt ist. Die Saugleitung 10' kann in einen Instrumentenkanal 13 einmünden, der für die Einführung von Arbeitsinstrumenten vorgesehen ist und mit einer Abdeckkappe 14 dicht verschließbar ist. Das in Fig. 1 dargestellte Trainingsendoskop ist dadurch gegenüber dem Original-Endoskop modifiziert, dass die distalseitige Saugleitung 10' durch einen Verschluß 15 verschlossen ist, so dass kein Durchfluss durch die distalseitige Saugleitung 10' stattfinden kann.

Das Spül- und Insufflationsventil 8 dient zum Steuern der Funktionen des Spülens und Insufflierens, d. h., des Einleitens einer Spülflüssigkeit bzw. eines Insufflationsgases in das Operationsgebiet. Hierzu sind in der Endoskopvorrichtung proximalseitig des Spül- und Insufflationsventils 8 eine Insufflationsleitung 11 und eine Spülleitung 12 vorgesehen, auf die das Spül- und Insufflationsventil 8 einwirkt. Distalseitig des Spül- und Insufflationsventils 8 ist ebenfalls eine Insufflationsleitung 11' vorgesehen, die bei einem Original-Endoskop durch den Endoskopschaft 4 bis zum distalen Ende 6 durchgeführt ist. Weiterhin ist distalseitig des Spül- und Insufflationsventils 8 eine Spülleitung 12' vorgesehen, die bei einem Original-Endoskop ebenfalls bis zum distalen Ende 6 des Endoskopschafts 4 durchgeführt ist. Die Insufflationsleitung 11' kann vor dem distalen Ende 6 in die Spülleitung 12' einmünden. Das in Fig. 1 dargestellte Trainingsendoskop ist gegenüber dem Original-Endoskop weiterhin dadurch modifiziert, dass die distalseitige Insufflationsleitung 11' durch einen Verschluß 16 verschlossen ist, so dass kein Durchfluss durch die distalseitige Insufflationsleitung 11' stattfinden kann, und dass die distalseitige Spülleitung 12' eine Unterbrechung 17 aufweist, so das ein durchströmendes Fluid dort entweichen kann. Das Entweichen des Fluids und ein entsprechender Druckabbau in der distalseitigen Spülleitung 12' wird dadurch ermöglicht, dass der Handgriff 3 nicht fluiddicht ausgebildet ist; die distalseitige Spülleitung 12' kann zu diesem Zweck aber auch im Bereich des Handgriffs 3 mit dem Außenraum verbunden sein (nicht dargestellt). Das Saugventil 7 und das Insufflations- und Spülventil 8 sind gegenüber einem Original-Endoskop nicht modifiziert.

Die Endoskopvorrichtung 2 kann ferner einen weiteren Instrumentenkanal 13' aufweisen, der durch eine Verschlusskappe 14' verschlossen werden kann. Darüber hinaus kann eine weitere Leitung 18, die bis zum distalen Ende 6 durchgeführt ist, vorhanden sein, sowie ggf. weitere, in Fig. 1 nicht dargestellte Komponenten eines klinisch einsetzbaren Endoskops, beispielsweise weitere Bedienungs- und Übertragungselemente zur Steuerung der Bewegung bzw. Abwinkelung des distalen Endbereichs des Schafts, eine BeleuchtungsOptik, eine Beobachtungs-Optik, eine Kamera oder ein Kamera-Dummy mit dazugehörigen Bedienungselementen.

Proximalseitig umfasst die Endoskopvorrichtung 2 einen Versorgungsschlauch 5, durch den die Leitungen 10, 11, 12 und 18 verlaufen und im proximalen Endbereich des Versorgungsschlauchs 5 jeweils zu Anschlüssen geführt sind. Die Saugleitung 10 ist über den Sauganschluss 20 mit der Versorgungseinheit 30 verbunden, wobei die Verbindung am Sauganschluss 20 der Endoskopvorrichtung 2 und ggf. an einem Sauganschluss 20' der Versorgungseinheit 30 lösbar ausgestaltet sein kann. Die Insufflationsleitung 11 ist über den Druckanschluss 21 lösbar mit der Versorgungseinheit 30 verbunden, wobei der Druckanschluss 21 Teil eines Steckers 22 ist, der dem Lichtanschlussstecker eines Original-Endoskops nachgebildet ist. Der Stecker umfasst einen Stift 23, der beim Original-Endoskop der Einkopplung des Beleuchtungslichts in die Beleuchtungsoptik des Endoskops dient. Der Stecker 22 mit dem Stift 23 passt in eine Buchse 24 der Versorgungseinheit 30, die der Lichtanschlussbuchse der Lichtquelle für ein Original-Endoskop nachgebildet ist. Die weitere Leitung 18 ist zu einem Anschluss 25 geführt, der als Luer-Lock ausgebildet sein kann.

Die Spülleitung 12 ist zu einem Spülanschluss 26 geführt, an den ein Spülflüssigkeitsbehälter 29, beispielsweise eine Spülflüssigkeitsflasche, angeschlossen ist. Der Spülanschluss 26 kann ebenfalls dem eines Original-Endoskops entsprechen und neben der Spülleitung 12 eine Druckleitung 27 umfassen, die im Bereich des proximalen Endes des Versorgungsschlauchs 5 von der Insufflationsleitung 11 abzweigt und beim Betrieb eines Original-Endoskops dazu dient, den Spülflüssigkeitsbehälter 29 mit Überdruck zu beaufschlagen, damit die Spülflüssigkeit durch das in diese eintauchende Ende der Spülleitung 12 in diese hineingedrückt wird. Das vom Anschluss 26 in den Spülflüssigkeitsbehälter 29 reichende Teilstück 12" der Spülleitung und die Druckleitung 27 können in einem Schlauch, der beide Leitungen enthält und mit der Endoskopvorrichtung am Anschluss 26 lösbar verbunden ist, zusammengefasst sein. Beim Betrieb des erfindungsgemäßen Simulationssystems 1 gemäß Ausführungsbeispiel ist der Spülflüssigkeitsbehälter 29 bevorzugt leer, d. h., lediglich mit Luft gefüllt. Die Druckleitung 27 ist durch einen Verschluss 28 verschlossen, und der Spülflüssigkeitsbehälter 29 ist über ein weiteres Teilstück 12'" der Spülleitung mit der Versorgungseinheit 30 verbunden.

Innerhalb der Versorgungseinheit 30 sind die Saugleitung 10, die Insufflationsleitung 11 und die Spülleitung 12'" mit einer Pumpeneinheit 31 verbunden, die eine Saugpumpe, eine Insufflationspumpe und eine Spülpumpe enthalten kann, die von einem gemeinsamen Antrieb angetrieben werden (nicht dargestellt). Die Saugpumpe beaufschlagt die Saugleitung 10 mit Unterdruck, die Insufflationspumpe die Insufflationsleitung 11 mit Überdruck und die Spülpumpe die Spülleitung 12 bzw. 12'" ebenfalls mit Überdruck, wobei die Drücke und Fördermengen möglichst denen beim klinischen Einsatz eines Original-Endoskops mit den entsprechenden Pumpen entsprechen.

An der Saugleitung 10, der Insufflationsleitung 11 und der Spülleitung 12'" ist jeweils ein Drucksensor 32, 32', 32" angeordnet zur Messung des Drucks in der jeweiligen Leitung. Die Messwerte werden in digitaler oder analoger Form über Signalleitungen 33, 33', 33" zu einer Vorverarbeitungseinheit 60 übertragen, von wo aus die vorverarbeiteten Messwerte über eine Schnittstelle 35 und ein Kabel 36 an die Steuerungseinrichtung 70 übertragen werden.

Die Steuerungseinrichtung 70 umfasst insbesondere einen Bildprozessor zur Erzeugung bzw. Bearbeitung eines Videobilds, das auf einer Anzeigeeinrichtung 71 für den Benutzer dargestellt wird. Das Videobild stellt als virtuelle Realität eine endoskopische Sicht des Körperhohlraums dar, der bei dem Training mit dem Simulationssystem einem endoskopischen Eingriff unterzogen wird. Hierfür kann die Steuerungseinrichtung 70 insbesondere Speichermittel umfassen, auf denen Daten über den betreffenden Körperhohlraum gespeichert sind und für die Simulation abgerufen werden können. Ferner kann der Steuerungseinrichtung 70 eine Eingabeeinrichtung 72 zugeordnet sein, die beispielsweise als Tastatur oder Touch-Schreen ausgebildet sein kann, und über die der Benutzer die Simulation definieren, starten und/oder steuern kann. Die Steuerungseinrichtung 70 kann beispielsweise als PC oder auch als spezieller Bildverarbeitungsrechner ausgebildet sein oder einen solchen umfassen. Ferner können weitere Eingabe- und/oder Anzeigeeinrichtungen vorgesehen sein. Das erfindungsgemäße Simulationssystem kann noch weitere Komponenten umfassen, wie beispielsweise eine Einrichtung zur Detektion von Bewegungen des distalen Endes 6 bzw. das Schafts 4, die bei der Erzeugung des virtuellen Bilds berücksichtigt werden.

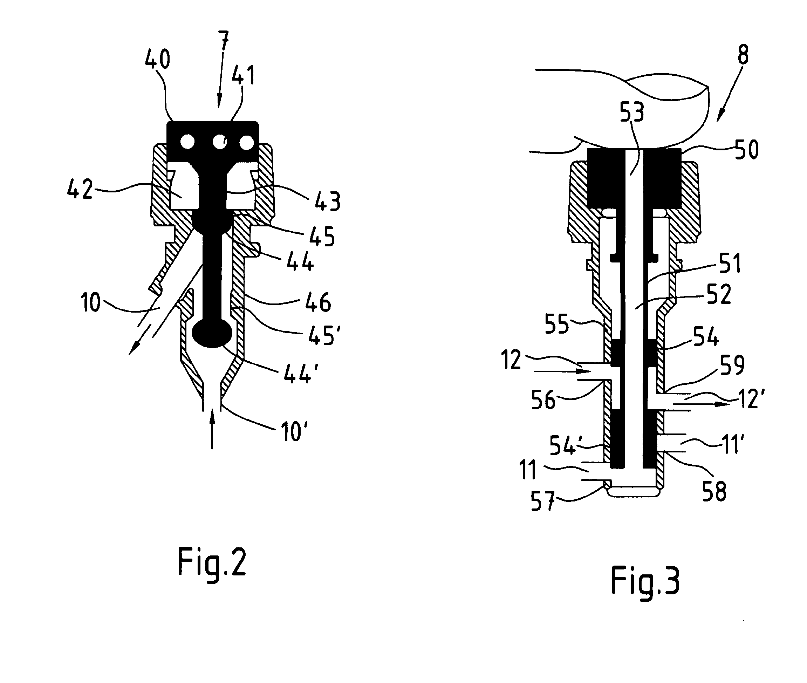

Das Saugventil 7 umfasst, wie aus Fig. 2 ersichtlich, einen Ventilknopf 40, der eine Mehrzahl von Querbohrungen 41 aufweisen kann, die mit einem Innenraum 42 des Ventils in Verbindung stehen. Im Ausgangszustand, der in Fig. 1 dargestellt ist, steht der Ventilknopf 40 soweit heraus, dass sich ein mit dem Ventilknopf 40 über einen Stößel 43 verbundener, näherungsweise kugelförmiger erster Absperrkörper 44 oberhalb eines ersten Ventilsitzes 45 befindet, so dass die Saugleitung 10 mit dem Innenraum 42 und von dort aus über die Bohrungen 41 oder auch direkt über den Zwischenraum zwischen Ventilgehäuse 46 und Ventilknopf 40 mit dem Außenraum verbunden ist. In diesem Zustand kann daher, wenn die Saugleitung 10 mit Unterdruck beaufschlagt wird, Luft ungehindert in diese einströmen. Andererseits ist in dieser Stellung des Saugventils 7 ein zweiter, ebenfalls näherungsweise kugelförmiger Absperrkörper 44', der ebenfalls über den Stößel 43 mit dem Ventilknopf 40 verbunden ist, in einen zweiten Ventilsitz 45' eingesenkt. Zu der distalseitigen Saugleitung 10' besteht daher keine Verbindung. Wird zur Betätigung des Saugventils 7 der Ventilknopf 40 und damit der Stößel 43 eingedrückt, wie in Fig. 2 dargestellt, so senkt sich der erste Absperrkörper 44 in den ersten Ventilsitz 45, so dass die Verbindung der Saugleitung 10 zum Außenraum unterbrochen wird. Andererseits hebt sich der zweite Absperrkörper 44' aus dem zweiten Ventilsitz 45' heraus, so dass eine Verbindung zur distalen Saugleitung 10' entsteht.

Das Insufflations- und Spülventil 8 umfasst, wie aus Fig. 3 ersichtlich, einen Ventilknopf 50, über den ein Stößel 51 betätigt werden kann. Ventilknopf 50 und Stößel 51 weisen eine zentrale Bohrung 52 auf, die am einen Ende mit der Insufflationsleitung 11 in Verbindung steht und am anderen Ende in einer Öffnung 53 in den Außenraum endet. Mit dem Stößel 51 sind nach Art eines Pumpventils im wesentlichen zylindrische Absperrkörper 54, 54' verbunden, die zum Öffnen und Verschließen der mit dem Insufflations- und Spülventil 8 verbundenen proximalseitigen Spülleitung 12, distalseitigen Insufflationsleitung 11' und distalseitigen Spülleitung 12' dienen. Im Ausgangszustand, der in Fig. 1 dargestellt ist, steht der Ventilknopf 50 soweit heraus, dass sich der erste Absperrkörper 54 oberhalb des Anschlusses 56 der Spülleitung 12 am Ventilgehäuse 55 befindet, und der zweite Absperrkörper 54' den Anschluss 59 der distalseitigen Spülleitung 12' verschließt. Solange die Öffnung 53 nicht verschlossen ist, kann ein in der Insufflationsleitung 11 befindliches, unter Überdruck stehendes Fluid ungehindert in den Außenraum entweichen, so dass sich in der Insufflationsleitung 11 kein wesentlicher Überdruck aufbauen kann. Wird die Öffnung 53 durch einen Finger verschlossen, wie in Fig. 3 angedeutet, kann sich dagegen in der Insufflationsleitung 11 Druck aufbauen. Wird das Insufflations- und Spülventil 8 durch weiteren Druck mit dem Finger eingedrückt, wie in Fig. 3 dargestellt, so wird der Anschluss 58 der distalseitigen Spülleitung 12' durch den zweiten Absperrkörper 54' freigegeben, und ein in der proximalseitigen Spülleitung 12 befindliches Fluid kann in die distalseitige Spülleitung 12' strömen. Da diese an der Unterbrechung 17 offen ist, kann sich in dieser Ventilstellung kein wesentlicher Druck in der Spülleitung 12 aufbauen. Das Insufflations- und Spülventil 8 lässt auch Zwischenstellungen zu, die durch Niederdrücken des Ventilknopfs 50 über einen Teil des Verstellwegs erzielt werden und die beispielsweise ein gleichzeitiges teilweises Abdecken der Anschlüsse 58 und 59 der distalseitigen Insufflationsleitung 11' bzw. Spülleitung 12' ermöglichen. Ebenso kann der erste Absperrkörper den Anschluss 56 der proximalseitigen Spülleitung 12 teilweise abdecken.

Die in Fig. 2 dargestellten Pfeile geben die Flussrichtung eines abgesaugten Mediums beim Saugbetrieb eines Original-Endoskops an, das ein derart ausgebildetes Saugventil aufweist. In Fig. 3 geben die Pfeile jeweils die Flussrichtung des Spülmediums an, wenn dieses von der Pumpeneinheit mit Überdruck beaufschlagt wird. Die Ventile 7, 8 können weiterhin Federn umfassen, die den jeweiligen Stößel bzw. Ventilknopf mit einer Rückstellkraft beaufschlagen, die beim Eindrücken der Ventilknöpfe überwunden werden muss und nach einer Betätigung die Ventilknöpfe in ihre Ausgangsstellung zurückbringt (nicht dargestellt).

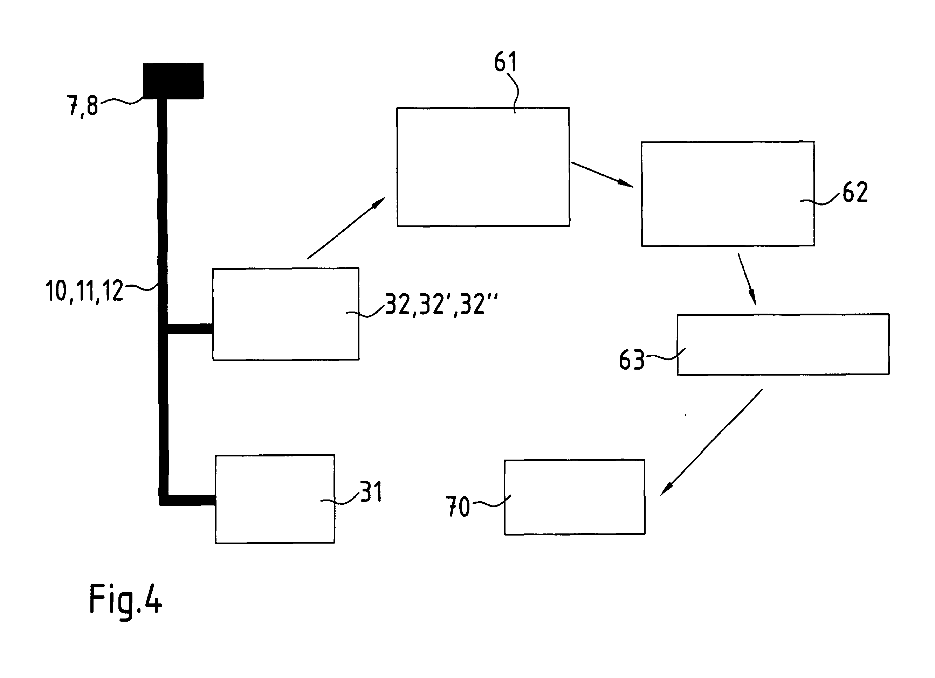

In Fig. 4 ist in schematischer Weise beispielhaft der Datenfluss von den Drucksensoren zur Steuerungseinrichtung 70 dargestellt. An die symbolisch dargestellte Saug-, Insufflationsoder Spülleitung 10, 11, 12, die von der Pumpeneinheit 31 zum Ventil 7, 8 führt, ist ein Drucksensor 32, 32', 32"an einer von der Leitung 10, 11, 12 abzweigenden Leitung angeordnet und steht dadurch in Fluidkommunikation mit dieser, um den Druck innerhalb der Leitung 10, 11, 12 zu messen. Die zunächst in analoger Form abgegebenen Druckmesswerte werden an einen Verstärker 61 übermittelt, von diesem an einen A/D-Wandler 62, und von diesem in digitaler Form an eine USB-Schnittstelle 63. Von hier aus erfolgt die Übertragung an die Steuerungseinrichtung 70, die beispielsweise ein PC sein kann. Verstärker 61, A/D-Wandler 62 und Schnittstelle 63 können in der Vorverarbeitungseinheit 60 zusammengefasst sein.

Bei der Durchführung einer Schulung bzw. eines Trainings für eine endoskopische Operation hält der Benutzer die Endoskopvorrichtung 2 am Handgriff 3, zunächst ohne die Ventile 7, 8 zu betätigen. Nach dem Einschalten der Pumpeneinheit 31 baut sich in der Saugleitung 10 noch kein wesentlicher Druck auf, da in der Ausgangsstellung des Saugventils 7 Luft in die Saugleitung 10 einströmen kann. Ebenso baut sich in der Insufflationsleitung 11 kein wesentlicher Druck auf, da die Öffnung 53 der Bohrung 52 noch offen ist. Lediglich in der Spülleitung 12, die durch das Insufflations- und Spülventil 8 verschlossen ist, baut sich entsprechend der Einstellung der Pumpeneinheit 31 für die Spülfunktion ein Überdruck auf, der von dem Sensor 32" gemessen wird; der Messwert wird über die Signalleitung 33" an die Vorverarbeitungseinheit 60 und von dort an die Steuerungseinrichtung 70 übertragen. Aufgrund des Strömungswiderstands in den Leitungen kann sich auch in der Saugleitung 10 und in der Insufflationsleitung 11 ein geringer Druckunterschied zum Außendruck aufbauen, der ggf. messbar sein kann. Die Steuerungseinrichtung 70 stellt anhand des Drucks in der Spülleitung 12 und ggf. des geringen Drucks in der Saugleitung 10 und in der Insufflationsleitung 11 fest, dass sich die Ventile 7 und 8 in Ausgangsstellung befinden, d. h., dass vom Benutzer keine der Funktionen Saugen, Insufflieren und Spülen betätigt worden ist. Dies wird bei der Berechnung des virtuellen Bildes entsprechend berücksichtigt.

Um die Insufflation zu beginnen, deckt der Benutzer die Öffnung 53 mit einem Finger ab. Nun kann sich in der Insufflationsleitung 11 ein Überdruck aufbauen, entsprechend der Einstellung der Pumpeneinheit 31 für die Insufflation. Dieser wird durch den Sensor 32' gemessen. Die Steuerungseinrichtung stellt anhand des übermittelten Druckwerts des Sensors 32' fest, dass der Benutzer die Insufflation initiiert hat, was bei der Erzeugung des virtuellen Bildes berücksichtigt wird. Strömungswiderstände, Volumina und Einstellungen sind bevorzugt derart gewählt, dass der Druckaufbau in ähnlicher Weise, ggf. allmählich, erfolgt wie bei einem Original-Endoskop in der betreffenden Operationssituation.

Drückt nun der Benutzer das Insufflations- und Spülventil 8 nach unten, so ändert sich am Druck in der Insufflationsleitung 11 nichts, da die distalseitige Insufflationsleitung sowieso durch den Verschluss 16 verschlossen ist. Andererseits wird nun eine Verbindung zwischen der proximalseitigen Spülleitung 12 und der distalseitigen Spülleitung 12' hergestellt, wo das in der Spülleitung 12 enthaltene Fluid durch die Unterbrechung 17 entweichen kann. Der in der Spülleitung 12 bzw. 12" aufgebaute Überdruck wird daher auf Null oder auf einen sehr geringen, durch den Strömungswiderstand bestimmten Wert zurückgehen. Auch hierbei können die Strömungswiderstände, Volumina und Einstellungen derart gewählt werden, dass der zeitliche Ablauf des Druckabbaus realitätsnah erfolgt. Die Steuerungseinrichtung 70 ermittelt aus dem Druckabfall in der Spülleitung 12", dass der Spülvorgang ausgelöst worden ist, was bei der Berechnung des virtuellen Bilds berücksichtigt wird.

Je nach simulierter Operation oder Aufgabenstellung kann natürlich auch ein anderer Ablauf als der oben beispielhaft beschriebene gewählt werden.

### Bezugszeichenliste

- 1: Simulationssystem
- 2: Endoskopvorrichtung
- 3: Handgriff
- 4: Schaft
- 5: Versorgungsschlauch
- 6: distales Ende
- 7: Saugventil
- 8: Spül- und Insufflationsventil
- 10, 10': Saugleitung
- 11, 11': Insufflationsleitung
- 12, 12', 12", 12'": Spülleitung
- 13, 13': Instrumentenkanal
- 14, 14': Verschlusskappe
- 15: Verschluss
- 16: Verschluss
- 17: Unterbrechung
- 18: Leitung
- 20, 20': Sauganschluss
- 21: Druckanschluss
- 22: Stecker
- 23: Stift
- 24: Buchse
- 25: Anschluss
- 26: Spülanschluss
- 27: Druckleitung
- 28: Verschluss
- 29: Spülflüssigkeitsbehälter
- 30: Versorgungseinheit
- 31: Pumpeneinheit
- 32, 32', 32": Drucksensor
- 33, 33', 33": Signalleitung
- 35: Schnittstelle
- 36: Kabel
- 40: Ventilknopf
- 41: Querbohrung
- 42: Innenraum
- 43: Stößel
- 44, 44': Absperrkörper
- 45, 45': Ventilsitz
- 46: Ventilgehäuse
- 50: Ventilknopf
- 51: Stößel
- 52: Bohrung
- 53: Öffnung
- 54,: 54' Absperrkörper
- 55: Ventilgehäuse
- 56: Anschluss
- 57: Anschluss
- 58: Anschluss
- 59: Anschluss
- 60: Vorverarbeitungseinheit
- 61: Verstärker
- 62: A/D-Wandler
- 63: USB-Schnittstelle
- 70: Steuerungseinrichtung
- 71: Anzeigeeinrichtung
- 72: Eingabeeinrichtung

## Patentansprüche

1. Simulationssystem für das Training endoskopischer Operationen, umfassend eine Endoskopvorrichtung (2), die ein Bedienteil mit mindestens einem Ventil (7, 8) aufweist, eine Steuerungseinrichtung (70) zur Erzeugung eines virtuellen Bildes einer endoskopischen Operationsszene in Abhängigkeit von einer Betätigung des mindestens einen Ventils (7, 8) und eine Anzeigeeinrichtung (71) zur Anzeige des virtuellen Bildes, **dadurch gekennzeichnet, dass** die Endoskopvorrichtung (2) mindestens eine Leitung (10, 11, 12) aufweist, auf die das mindestens eine Ventil (7, 8) einwirkt, und dass das Simulationssystem ferner Druckerzeugungsmittel umfasst zur Beaufschlagung der mindestens einen Leitung (10, 11, 12) mit Unter- oder Überdruck, Sensormittel zur Messung von Druck und/oder Fluss in der mindestens einen Leitung (10, 11, 12) und Übertragungsmittel zur Übertragung der von den Sensormitteln gelieferten Messwerte an die Steuerungseinrichtung (70) zur Verwendung bei der Erzeugung des virtuellen Bildes.

2. Simulationssystem nach Anspruch 1, **dadurch gekennzeichnet, dass** das Bedienteil eine Mehrzahl von Ventilen (7, 8) aufweist und dass die Endoskopvorrichtung (2) eine Saugleitung (10), eine Insufflationsleitung (11) und eine Spülleitung (12) aufweist, auf die die Ventile (7, 8) einwirken.

3. Simulationssystem nach Anspruch 1 oder 2, **dadurch gekennzeichnet, dass** die Sensormittel Drucksensoren (32, 32', 32") sind.

4. Simulationssystem nach Anspruch 2 oder 3, **dadurch gekennzeichnet, dass** die Druckerzeugungsmittel zur Erzeugung von Unterdruck in der Saugleitung (10) und zur Erzeugung von Überdruck in der Insufflationsleitung (11) und in der Spülleitung (12) ausgebildet sind.

5. Simulationssystem nach einem der vorhergehenden Ansprüche, **dadurch gekennzeichnet, dass** die Druckerzeugungsmittel Pumpen, insbesondere Rollen- oder Membranpumpen sind.

6. Simulationssystem nach einem der vorhergehenden Ansprüche, **dadurch gekennzeichnet, dass** die Sensormittel analoge Sensoren umfassen und dass die Übertragungsmittel einen Verstärker (61), einen A/D-Wandler (62) und eine USB-Schnittstelle (63) umfassen.

7. Simulationssystem nach einem der vorhergehenden Ansprüche, **dadurch gekennzeichnet, dass** die Druckerzeugungsmittel und die Sensormittel in einer Versorgungseinheit (30) zusammengefasst sind.

8. Simulationssystem nach Anspruch 7, **dadurch gekennzeichnet, dass** die Endoskopvorrichtung (2) mit der Versorgungseinheit (30) lösbar verbunden ist.

9. Simulationssystem nach einem der vorhergehenden Ansprüche, **dadurch gekennzeichnet, dass** die Endoskopvorrichtung (2) ein flexibles Endoskop mit einem Handgriff (3), einem flexiblen, langerstreckten Schaft (4) und einem Versorgungsschlauch (5) ist und einen Sauganschluss (20), einen Druckanschluss (21) und einen Spülanschluss (26) aufweist.

10. Simulationssystem nach einem der vorhergehenden Ansprüche, **dadurch gekennzeichnet, dass** die Endoskopvorrichtung (2) ein klinisch einsetzbares Endoskop ist.

11. Simulationssystem nach einem der Ansprüche 1 bis 9, **dadurch gekennzeichnet, dass** die Endoskopvorrichtung (2) ein Trainingsendoskop ist.

12. Simulationssystem nach einem der Ansprüche 9 bis 11, **dadurch gekennzeichnet, dass** die Sensormittel in den Anschlüssen (20, 21, 26) des Endoskops bzw. des Trainingsendoskops angeordnet sind.

13. Simulationssystem nach einem der Ansprüche 2 bis 12, **dadurch gekennzeichnet, dass** die Saugleitung (10') distalseitig des auf die Saugleitung einwirkenden Ventils (7) und die Insufflationsleitung (11') distalseitig des auf die Insufflationsleitung einwirkenden Ventils (8) verschließbar sind.

14. Simulationssystem nach einem der Ansprüche 2 bis 13, **dadurch gekennzeichnet, dass** die Saugleitung (10') distalseitig des auf die Saugleitung einwirkenden Ventils (7) und die Insufflationsleitung (11') distalseitig des auf die Insufflationsleitung einwirkenden Ventils (8) verschlossen sind und die Spülleitung (12') distalseitig des auf die Spülleitung einwirkenden Ventils (8) offen ist.

15. Verfahren für das Training endoskopischer Operationen, umfassend folgende Schritte:
- Bereitstellen einer Endoskopvorrichtung (2), die ein Bedienteil mit mindestens einem Ventil (7, 8) und mindestens eine Leitung (10, 11, 12) aufweist, auf die das mindestens eine Ventil (7, 8) einwirkt,
- Beaufschlagen der mindestens einen Leitung (10, 11, 12) mit Unter- oder Überdruck,
- Erfassen einer Betätigung des mindestens einen Ventils (7, 8) durch Messung von Druck und/oder Fluss in der mindestens einen Leitung (10, 11, 12),
- Übertragen der Messwerte von Druck und/oder Fluss an eine Steuerungseinrichtung (70),
- Erzeugen eines virtuellen Bildes einer endoskopischen Operationsszene durch die Steuerungseinrichtung (70) in Abhängigkeit von der durch die Messwerte erfassten Betätigung der Ventile (7, 8) und
- Anzeigen des virtuellen Bildes für einen Benutzer.

## Claims

1. Simulation system for training endoscopic operations, comprising an endoscope device (2) which has an operating part with at least one valve (7, 8), a control apparatus (70) for producing a virtual image of an endoscopic operation scene depending on an actuation of the at least one valve (7, 8) and a display apparatus (71) for displaying the virtual image, **characterized in that** the endoscope device (2) has at least one line (10, 11, 12), onto which the at least one valve (7, 8) acts, and **in that** the simulation system furthermore comprises pressure producing means for applying negative or positive pressure to the at least one line (10, 11, 12), sensor means for measuring pressure and/or flow in the at least one line (10, 11, 12) and transmission means for transmitting the measurement values supplied by the sensor means to the control apparatus (70) for use when producing the virtual image.

2. Simulation system according to Claim 1, **characterized in that** the operating part has a plurality of valves (7, 8) and **in that** the endoscope device (2) has a suction line (10), an insufflation line (11) and a rinsing line (12), on which the valves (7, 8) act.

3. Simulation system according to Claim 1 or 2, **characterized in that** the sensor means are pressure sensors (32, 32', 32").

4. Simulation system according to Claim 2 or 3, **characterized in that** the pressure production means are embodied to produce negative pressure in the suction line (10) and to produce positive pressure in the insufflation line (11) and in the rinsing line (12).

5. Simulation system according to one of the preceding claims, **characterized in that** the pressure production means are pumps, in particular roller pumps or diaphragm pumps.

6. Simulation system according to one of the preceding claims, **characterized in that** the sensor means comprise analogue sensors and **in that** the transmission means comprise an amplifier (61), an analogue-to-digital converter (62) and a USB interface (63).

7. Simulation system according to one of the preceding claims, **characterized in that** the pressure production means and the sensor means are combined in a supply unit (30).

8. Simulation system according to Claim 7, **characterized in that** the endoscope device (2) is detachably connected to the supply unit (30).

9. Simulation system according to one of the preceding claims, **characterized in that** the endoscope device (2) is a flexible endoscope with a handle (3), a flexible, elongate shank (4) and a supply tube (5) and has a suction connector (20), a pressure connector (21) and a rinsing connector (26).

10. Simulation system according to one of the preceding claims, **characterized in that** the endoscope device (2) is an endoscope employable in a clinical context.

11. Simulation system according to one of Claims 1 to 9, **characterized in that** the endoscope device (2) is a training endoscope.

12. Simulation system according to one of Claims 9 to 11, **characterized in that** the sensor means are arranged in the connectors (20, 21, 26) of the endoscope or of the training endoscope.

13. Simulation system according to one of Claims 2 to 12, **characterized in that** the suction line (10') is sealable on the distal side of the valve (7) acting on the suction line and the insufflation line (11') is sealable on the distal side of the valve (8) acting on the insufflation line.

14. Simulation system according to one of Claims 2 to 13, **characterized in that** the suction line (10') is sealed on the distal side of the valve (7) acting on the suction line and the insufflation line (11') is sealed on the distal side of the valve (8) acting on the insufflation line and the rinsing line (12') is open on the distal side of the valve (8) acting on the rinsing line.

15. Method for training endoscopic operations, comprising the following steps:
- providing an endoscope device (2) which has an operating part with at least one valve (7, 8) and at least one line (10, 11, 12), onto which the at least one valve (7, 8) acts,
- applying negative or positive pressure to the at least one line (10, 11, 12),
- registering an actuation of the at least one valve (7, 8) by measuring pressure and/or flow in the at least one line (10, 11, 12),
- transmitting the measurement values of pressure and/or flow to a control apparatus (70),
- producing a virtual image of an endoscopic operation scene by the control apparatus (70), depending on the actuation of the valves (7, 8) registered by means of the measurement values, and
- displaying the virtual image to a user.

## Revendications

1. Système de simulation pour l'entraînement d'opérations endoscopiques, comprenant un dispositif d'endoscope (2), qui présente une partie de conduite avec au moins une soupape (7, 8), un dispositif de commande (70) pour la production d'une image virtuelle d'une scène d'opération endoscopique en fonction d'un actionnement de ladite au moins une soupape (7, 8), et un dispositif d'affichage (71) pour l'affichage de l'image virtuelle, **caractérisé en ce que** le dispositif d'endoscope (2) présente au moins une conduite 10, 11, 12), sur laquelle ladite au moins une soupape (7, 8) agit, et **en ce que** le système de simulation comprend en outre des moyens de production de pression pour soumettre ladite au moins une conduite (10, 11, 12) à une dépression ou à une surpression, des moyens de détection pour mesurer la pression et/ou le débit dans ladite au moins une conduite (10, 11, 12) et des moyens de transmission pour la transmission des valeurs de mesure fournies par les moyens de détection au dispositif de commande (70) en vue de leur utilisation lors de la production de l'image virtuelle.

2. Système de simulation selon la revendication 1, **caractérisé en ce que** la partie de conduite présente une multiplicité de soupapes (7, 8) et **en ce que** le dispositif d'endoscope (2) présente une conduite d'aspiration (10), une conduite d'insufflation (11) et une conduite de purge (12), sur lesquelles les soupapes (7, 8) agissent.

3. Système de simulation selon la revendication 1 ou 2, **caractérisé en ce que** les moyens de détection sont des capteurs de pression (32, 32', 32").

4. Système de simulation selon la revendication 2 ou 3, **caractérisé en ce que** les moyens de production de pression sont configurés pour produire une dépression dans la conduite d'aspiration (10) et pour produire une surpression dans la conduite d'insufflation (11) et dans la conduite de purge (12).

5. Système de simulation selon l'une quelconque des revendications précédentes, **caractérisé en ce que** les moyens de production de pression sont des pompes, en particulier des pompes à rouleaux ou des pompes à membrane.

6. Système de simulation selon l'une quelconque des revendications précédentes, **caractérisé en ce que** les moyens de détection comprennent des détecteurs analogiques et **en ce que** les moyens de transmission comprennent un amplificateur (61), un convertisseur A/D (62) et une interface USB (63).

7. Système de simulation selon l'une quelconque des revendications précédentes, **caractérisé en ce que** les moyens de production de pression et les moyens de détection sont rassemblés dans une unité d'alimentation (30).

8. Système de simulation selon la revendication 7, **caractérisé en ce que** le dispositif d'endoscope (2) est relié de façon séparable à l'unité d'alimentation (30).

9. Système de simulation selon l'une quelconque des revendications précédentes, **caractérisé en ce que** le dispositif d'endoscope (2) est un endoscope flexible avec une poignée (3), une tige allongée flexible (4) et un tuyau d'alimentation (5) et présente un raccord d'aspiration (20), un raccord de pression (21) et un raccord de purge (26).

10. Système de simulation selon l'une quelconque des revendications précédentes, **caractérisé en ce que** le dispositif d'endoscope (2) est un endoscope utilisable en clinique.

11. Système de simulation selon l'une quelconque des revendications 1 à 9, **caractérisé en ce que** le dispositif d'endoscope (2) est un endoscope d'entraînement.

12. Système de simulation selon l'une quelconque des revendications 9 à 11, **caractérisé en ce que** les moyens de détection sont disposés dans les raccords (20, 21, 26) de l'endoscope ou de l'endoscope d'entraînement.

13. Système de simulation selon l'une quelconque des revendications 2 à 12, **caractérisé en ce que** la conduite d'aspiration (10') peut être fermée du côté distal de la soupape (7) agissant sur la conduite d'aspiration et la conduite d'insufflation (11') peut être fermée du côté distal de la soupape (8) agissant sur la conduite d'insufflation.

14. Système de simulation selon l'une quelconque des revendications 2 à 13, **caractérisé en ce que** la conduite d'aspiration (10') est fermée du côté distal de la soupape (7) agissant sur la conduite d'aspiration et la conduite d'insufflation (11') est fermée du côté distal de la soupape (8) agissant sur la conduite d'insufflation et la conduite de purge (12') est ouverte du côté distal de la soupape (8) agissant sur la conduite de purge.

15. Procédé pour l'entraînement d'opérations endoscopiques, comprenant les étapes suivantes:
- procurer un dispositif d'endoscope (2), qui présente une partie de conduite avec au moins une soupape (7, 8) et au moins une conduite (10, 11, 12), sur laquelle ladite au moins une soupape (7, 8) agit,
- soumettre ladite au moins une conduite (10, 11, 12) à une dépression ou une surpression,
- détecter un actionnement de ladite au moins une soupape (7, 8) par mesure de la pression et/ou du débit dans ladite au moins une conduite (10, 11, 12),
- transmettre les valeurs de mesure de la pression et/ou du débit à un dispositif de commande (70),
- produire une image virtuelle d'une scène d'opération endoscopique par le dispositif de commande (70) en fonction de l'actionnement des soupapes (7, 8) détecté par les valeurs de mesure, et
- afficher l'image virtuelle pour un utilisateur.
